# EUROPÄISCHE PATENTANMELDUNG

(11) **EP 1 386 960 A2**
(43) Veröffentlichungstag der Anmeldung: **04.02.2004**
(21) Anmeldenummer: 03016775.3
(22) Anmeldetag: 23.07.2003
(51) Int. Cl.: C12M 1/36, C12P 19/14

(54) **Verfahren und Vorrichtung zur kontinuierlichen Verarbeitung von Stärkematerial**

(30) Priorität: 03.08.2002 DE 10235715
(71) Anmelder: CAVITRON v.Hagen & Funke GmbH, 45549 Sprockhövel (DE)
(72) Erfinder: Fisch, Klaus, 58285 Gevelsberg (DE)
(74) Vertreter: Selting, Günther, Dipl.-Ing.

(57) **Zusammenfassung**

Bei der Herstellung von Leim oder Klebstoff wird Stärke aus einem Silo (10) in einen Dispergierbehälter (12) eingeführt und zu einer Suspension vermischt. Gleichzeitig wird ein Enzym zugegeben. In einer Aktivierungsvorrichtung (17) wird das Enzym durch Dampf aktiviert und die Suspension wird anschließend einem Verweilbehälter (20) zugeführt, der eine Aktivierungsstrecke (22) enthält. Das Produkt verlässt den Verweilbehälter (20) nach einer vorgesehenen Laufzeit. Anschließend erfolgt eine Deaktivierung des Enzyms in einer Deaktivierungsvorrichtung (26). Bei Beendigung der Materialzufuhr zu der Aktivierungsstrecke (22), z.B. bei Betriebsunterbrechung, werden mehrere Auslässe (A₀-A₄), die in unterschiedlichen Höhen an dem Verweilbehälter (20) vorgesehen sind, zeitlich gestaffelt geöffnet. Dadurch wird erreicht, dass in jeder Höhe das Material jeweils dann abgelassen wird, wenn es die vorgesehene Verweildauer erreicht hat.

## Beschreibung

Die Erfindung betrifft eine Vorrichtung zur kontinuierlichen Verarbeitung von Stärkematerial sowie ein Verfahren zum Beendigen einer kontinuierlich ablaufenden enzymatischen Aktivierungsreaktion.

Bei der Herstellung von Leim oder Klebstoff wird eine wässrige Stärkesuspension mit einem Additiv-Enzym versetzt. Durch eine Temperaturbehandlung der Suspension wird das Enzym aktiviert. Das Enzym hat die Wirkung, dass es während der Aktivierungsdauer die langkettigen Stärkemoleküle in kürzere Abschnitte aufspaltet. Die Aktivierung erfolgt in einem Verweilbehälter, der von der Suspension in einer vorgesehenen Laufzeit durchlaufen wird. Die Aktivierungsreaktion wird anschließend durch eine Deaktivierungsreaktion beendet. Bei der Deaktivierung wird das Enzym einer Hochtemperatur-Dampfbehandlung ausgesetzt. Anschließend wird das so verarbeitete Material einem Speicherbehälter zugeführt, in dem es zwischengespeichert wird, bevor es einer Weiterverarbeitungsmaschine zugeführt wird. Ein Anwendungsgebiet derartiger Verfahren ist die Papierherstellung. Hierbei werden Papierbahnen mit hoher Durchlaufgeschwindigkeit kontinuierlich erzeugt oder beschichtet. Dazu muss der erforderliche Leim zeitkonform hergestellt und zur Verfügung gehalten werden. Schwierigkeiten entstehen,wenn die weiterverarbeitende Maschine beispielsweise wegen eines Zerreißens der Papierbahn angehalten werden muss. Wird dann auch der kontinuierliche Prozess der Leimherstellung angehalten, so bleibt ein Teil des Stärkematerials zu lange in dem Verweilbehälter, was zur Folge hat, dass dieses Material nicht der gewünschten Qualität entspricht. Dadurch entsteht eine Materialmenge, die irgendwann später bei der Verarbeitungsmaschine ankommt und die Qualität des Endprodukts beeinträchtigt.

Der Erfindung liegt die Aufgabe zugrunde, eine Vorrichtung und ein Verfahren anzugeben, um bei einer enzymatischen Reaktion auch im Falle einer Beendigung oder Unterbrechung der Materialzufuhr sicherzustellen, dass das den Verweilbehälter verlassende Material die gewünschte Qualität hat.

Die Lösung dieser Aufgabe erfolgt bei der Vorrichtung mit den Merkmalen des Patentanspruchs 1 und bei dem Verfahren mit dem Merkmalen des Patentanspruchs 6.

Erfindungsgemäß ist vorgesehen, dass die Aktivierungsstrecke, in der die Aktivierungsreaktion des Enzyms abläuft, mehrere Auslässe aufweist und dass eine Steuerung vorgesehen ist, die im Falle einer Beendigung der Materialzufuhr die Auslässe entsprechend der ihnen zugeordneten Restlaufzeit zeitlich gestaffelt öffnet. Das Öffnen der Auslässe erfolgt derart, dass an jeder Stelle das Material jeweils dann abgelassen wird, wenn es die vorgesehene Verweildauer innerhalb definierter Grenzen erreicht hat.

Der Verweilbehälter bildet eine Laufzeitstrecke, die von dem Stärkematerial durchlaufen wird. Normalerweise erfolgt dies in der vorgesehenen Laufzeit. Wird nun der Materialtransport durch die Laufzeitstrecke unterbrochen, dann werden die im Zuge der Laufzeitstrecke angeordneten Auslässe gestaffelt geöffnet, so dass das Material, das sich an einer Stelle der Laufzeitstrecke befindet, die vorgesehene Verweildauer hat und dann aus dem Verweilbehälter entlassen wird. Damit wird eine rechtzeitige Beendigung der Aktivierungsreaktion sichergestellt und das auf diese Weise verarbeitete Stärkematerial hat die gewünschte Konsistenz.

Die Erfindung bietet den Vorteil einer gleichmäßigen Materialqualität auch im Falle der Beendigung oder Unterbrechung des Verarbeitungsprozesses. Die Folge hiervon ist, dass ein nachgeordneter Speicherbehälter, in dem das Material gespeichert und gemischt wird, zum Ausgleich etwaiger Materialschwankungen ein erheblich kleineres Volumen haben kann.

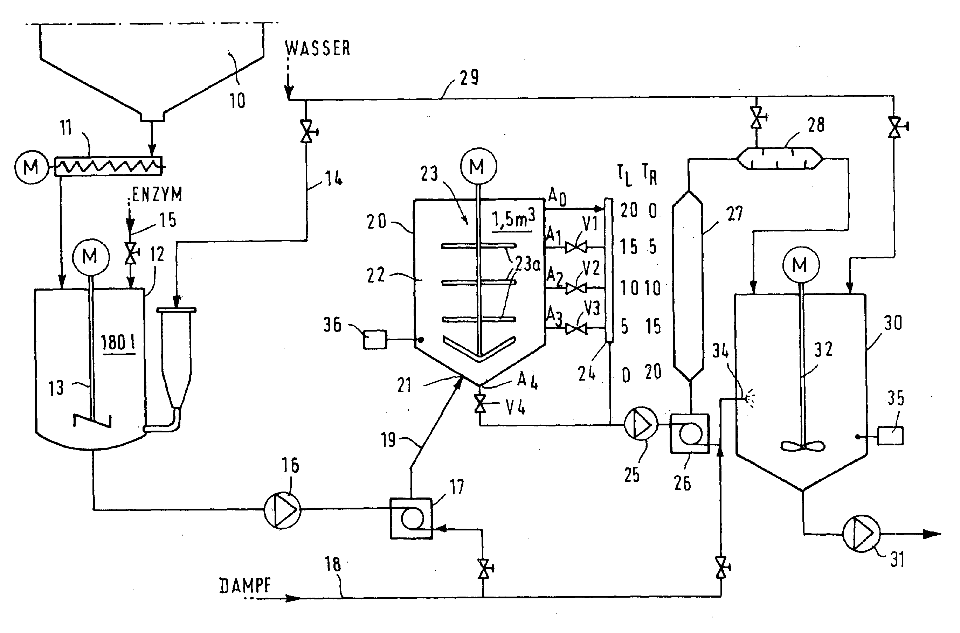

Im Folgenden wird unter Bezugnahme auf die einzige Figur der Zeichnung ein Ausführungsbeispiel der Erfindung näher erläutert, wobei das Ausführungsbeispiel nicht dahingehend zu verstehen ist, dass dadurch die Patentansprüche eingeschränkt würden.

In der Zeichnung ist eine Anlage zur kontinuierlichen Verarbeitung von Stärkematerial schematisch dargestellt. Die Anlage dient der Aufbereitung von Stärkematerial zur Erzeugung von Leim für mehrere Papierverarbeitungsmaschinen.

Die Anlage. dient zur Herstellung von Leim bzw. Klebstoff mit unterschiedlichen Stärkekonzentrationen. Dazu wird technische Stärke in Wasser dispergiert und mit einem Additiv-Enzym gemischt und anschließend gekocht.

Das Stärkepulver wird über Tankwagen in ein Silo 10 eingegeben und aus dem Silo mit einer motorgetriebenen Dosierschnecke 11 entnommen. Diese fördert die Stärke kontinuierlich in einen Dispergierbehälter 12, der ein motorgetriebenes Rührwerk 13 enthält. Dem Dispergierbehälter 12 wird über eine Leitung 14 in kontrollierter Form Wasser zugeführt. Außerdem wird in den Dispergierbehälter über eine Leitung 15 ein Enzym eingegeben, z.B. OC-Amylase. Die Stärke, z.B. Maisstärke, besteht aus langkettigen Glukosemolekülen. Das Enzym ist zunächst noch im inaktiven Zustand.

Im Dispergierbehälter wird die Dispersion über ein Schwimmerventil auf einem definierten Füllstand gehalten. Eine Pumpe 16 fördert die Dispersion zu einer Aktivierungsvorrichtung 17, in der das Material mittels Direktdampf auf die gewünschte Aktivierungstemperatur von etwa 80°C aufgeheizt wird. Der Dampf wird über eine Dampfleitung 18 in kontrollierter Form zugeführt. Der hierzu erforderliche Regelkreis ist aus Gründen der Übersicht nicht dargestellt. Von der Aktivierungsvorrichtung 17 führt eine Leitung 19 in den Verweilbehälter 20. Der Einlass 21 befindet sich in der Nähe des unteren Behälterendes. Der Verweilbehälter 20 bildet eine senkrecht verlaufende Aktivierungsstrecke 22, in der das Material von unten nach oben in geschichteter Form aufsteigt, ohne dass eine wesentliche vertikale Durchmischung erfolgt. Eine in dem Verweilbehälter vorgesehene motorisch angetriebene Mischvorrichtung 23 enthält Mischflügel 23a, die das Material nur horizontal umrühren, aber keine vertikale Umschichtung bewirken. Der Verweilbehälter 20, dem die Dispersion mit dem aktivierten Enzym zugeführt wird, ist mit einer thermischen Isolierung versehen, wodurch die Reaktionstemperatur von etwa 80°C über die gesamte Aktivierungsstrecke aufrechterhalten wird. Bei dieser Aktivierungstemperatur ist das Enzym aktiv. Es zerschneidet die Molekülketten des Stärkematerials auf eine gewünschte Länge. Nach einer vorgegebenen Zeit, die hier beispielsweise 20 Minuten beträgt, muss dieser Vorgang beendet und das Enzym deaktiviert werden.

Der Verweilbehälter 20 ist nahe seinem oberen Ende mit einem Auslass A₀ versehen, der den Betriebsauslass bildet. Der Auslass besteht aus einem von dem Verweilbehälter seitlich abgehenden Rohr, das ständig geöffnet ist.

Darunter befindet sich ein weiterer Auslass A₁ mit einem Ventil V₁. Darunter befinden sich weitere Auslässe A₂ und A₃ mit jeweils einem Ventil V₂ und V₃. Am unteren Behälterende befindet sich ein Auslass A₄ mit einem Ventil V₄.

Sämtliche Auslässe sind mit einer vertikalen Sammelleitung 24 verbunden. Die Sammelleitung 24 führt über eine Pumpe 25 zu einer Deaktivierungsvorrichtung 26. Darin wird das Produkt mittels Direktdampf, der von der Dampfleitung 18 geliefert wird, auf eine Inaktivierungstemperatur von etwa 120°C aufgeheizt. In einer angeschlossenen Inaktivierungsstrecke 27 wird das Produkt über mindestens zwei Minuten bei einer Lösetemperatur von etwa 120°C gehalten. Dadurch wird das Enzym zerstört und unwirksam gemacht. Das Stärkematerial wird in dem bisherigen Zustand stabilisiert.

In einem Statikmischer 28 erfolgt eine Nachverdünnung des Produktes mit Wasser auf die erforderliche Konzentration des Endproduktes. Die Wasserzugabe erfolgt geregelt, so dass sich eine gewünschte Konzentration einstellt.

Das Produkt wird anschließend einem Speicherbehälter 30 zugeführt, in dem es bei einer bestimmten Mindesttemperatur von ca. 70°C bereitgehalten wird, um anschließend durch eine Pumpe 31 einem Verbraucher zugeführt zu werden. Der Speicherbehälter 30 enthält eine Rührvorrichtung 32, eine Wasserzuführvorrichtung 33, eine Dampfzuführvorrichtung 34 und einen Füllstandssensor 35.

Die Pumpen 16,25 und 31 sind drehzahlgeregelt. Ebenso sind die Ventile, die an die Dampfleitung 18 bzw. die Wasserleitung 29 angeschlossen sind, Mengenstromventile, die in Abhängigkeit von Messwerten der Anlage gesteuert sind.

Bei kontinuierlichem Betrieb der Anlage wird in dem Dispergierbehälter 12 eine Dispersion erzeugt, die eine hohe Viskosität hat, d.h. sehr zähflüssig ist. Durch den Aufschluss in der Aktivierungsvorrichtung 17 und der Aktivierungsstrecke 22 wird das Produkt dünnflüssiger, wobei es normalerweise über den obersten Auslass A₀, der einen Überlauf bildet, abläuft. In diesem Zustand sind alle übrigen Auslässe A₁,A₂,A₃ und A₄ geschlossen. Die Endkonzentration der Paste, die die Anlage verlässt, beträgt etwa 10 %.

Die Mischvorrichtung 23 im Verweilbehälter 20 enthält eine rotierende Welle mit Mischflügeln 23a, die so ausgebildet sind, dass sie das Produkt in horizontaler Richtung umverteilen, jedoch keine wesentliche Bewegung des Produkts in vertikaler Richtung erzeugen. Dies bedeutet, dass in dem Verweilbehälter 20 eine Schichtung eintritt, wobei die übereinander liegenden Schichten eine Verweildauer haben, die von oben nach unten abnimmt. Die Verweildauer oder Laufzeit der Schichten, die sich in Höhe der Auslässe A₀-A₄ befinden, ist in der Zeichnung mit T_{L} bezeichnet und eingetragen. Beispielsweise beträgt die Laufzeit desjenigen Materials, das den obersten Auslass A₀ erreicht hat, 20 Minuten. Dasjenige Material, das den Auslass A₁ erreicht hat, hat eine Laufzeit von 15 Minuten hinter sich, das Material in Höhe des Auslasses A₂ von 10 Minuten und das Material am Auslass A₃ von 5 Minuten.

In der Zeichnung stellt T_{R} die Restlaufzeit dar, die das Produkt bei Erreichen der betreffenden Höhe noch in dem Verweilbehälter zubringen muss (in Minuten).

Wenn der Betrieb der Anlage unterbrochen oder beendet wird, wird unter anderem auch die Pumpe 16 stillgesetzt. Dies bedeutet, dass kein Produkt mehr durch den Einlass 21 in den Verweilbehälter 20 eintritt. Würde der Betrieb mit dem offenen Auslass A₀ fortgesetzt, während alle übrigen Auslässe A₁-A₄ geschlossen sind, würde der Aktivierungsprozess in der Aktivierungsstrecke 22 fortgesetzt werden, wodurch das Produkt dünnflüssig und unbrauchbar würde. Bei einem Stillsetzen der Pumpe 16 wird nach einer Zeit, die der Restlaufzeit T_{R} entspricht, das jeweilige Ventil V₁, V₂, V₃ oder V₄ geöffnet. Dies bedeutet, dass der Auslass A₁ nach 5 Minuten geöffnet wird, der Auslass A₂ nach 10 Minuten, der Auslass A₃ nach 15 Minuten und der Auslass A₄ nach 20 Minuten. Auf diese Weise wird sichergestellt, dass das Material, das sich in der jeweiligen Höhe im Verweilbehälter 20 befindet, nach einer Verweilzeit von 20 Minuten zur Deaktivierung abgeführt wird.

Der Dispergierbehälter ist ein relativ kleiner Behälter mit. einem Volumen von 180 1. Der Verweilbehälter 20 hat ein Volumen von 1,5 m³. Dieses Volumen ist so bemessen, dass der maximale Bedarf der Anlage unter Berücksichtigung der erforderlichen Laufzeit oder Verweildauer gedeckt werden kann. Das Volumen des Speicherbehälters 30 beträgt 5,0 m³. Es ist im Wesentlichen durch das benötigte Puffervolumen bestimmt. Der Speicherbehälter 30 braucht nicht so groß bemessen zu sein, dass Qualitätsabweichungen des zugeführten Materials verringert werden.

Die Steuerung der Auslässe A₀,A₁,A₂,A₃,A₄ des Verweilbehälters 20 erfolgt durch eine Zeitsteuereinrichtung, wobei die jeweilige Pegelstandshöhe durch einen Füllstandssensor 36 überwacht wird.

## Patentansprüche

1. Vorrichtung zur kontinuierlichen Verarbeitung von Stärkematerial, mit
einem Dispergierbehälter (12), in dem ein Enzym in die Stärke eingemischt wird,
einer Aktivierungsvorrichtung (17) zur Aktivierung des in der Stärke enthaltenen Enzyms, und
einem Verweilbehälter (20), der eine Aktivierungsstrecke (22) bildet,
**dadurch gekennzeichnet ,**
**dass** die Aktivierungsstrecke (22) mehrere Auslässe (A₀-A₄) an Stellen, die bei gleichmäßiger Materialzufuhr unterschiedlichen Laufzeiten (T_{L}) entsprechen, aufweist und dass eine Steuereinrichtung vorgesehen ist, die im Falle einer Beendigung der Materialzufuhr die Auslässe (A₀-A₄) entsprechend der ihnen zugeordneten Restlaufzeit (T_{R}) zeitlich gestaffelt öffnet.

2. Vorrichtung nach Anspruch 1, **gekennzeichnet durch** eine im Materialfluss hinter dem Verweilbehälter (20) angeordnete Deaktivierungsvorrichtung (26).

3. Vorrichtung nach Anspruch 1 oder 2, **dadurch gekennzeichnet, dass** ein Speicherbehälter (30) zur Aufnahme des verarbeiteten Stärkematerials vorgesehen ist, der eine Rührvorrichtung (32) und eine Wasserzuführvorrichtung (33) aufweist.

4. Vorrichtung nach einem der Ansprüche 1-3, **dadurch gekennzeichnet, dass** der Verweilbehälter (20) einen Materialeinlass (21) im unteren Bereich aufweist, und dass die Auslässe (A₀-A₄) in unterschiedlichen Höhen angeordnet sind.

5. Vorrichtung nach einem der Ansprüche 1-4, **dadurch gekennzeichnet, dass** die Auslässe (A₀-A₄) des Verweilbehälters (20) durch eine Sammelleitung (24) verbunden sind.

6. Verfahren zum Beendigen oder Unterbrechen einer kontinuierlich ablaufenden enzymatischen Aktivierungsreaktion, bei welcher ein mit dem Enzym vermischtes Material eine Aktivierungsstrecke (22) durchläuft, die eine bestimmte Verweildauer bewirkt,
**dadurch gekennzeichnet ,**
**dass** bei Beendigung der Materialzufuhr zu der Aktivierungsstrecke (22) mehrere im Verlauf der Aktivierungsstrecke vorgesehene Auslässe (A₀-A₄) zeitlich gestaffelt geöffnet werden, derart, dass an jeder Stelle das Material jeweils dann abgelassen wird, wenn es die vorgesehene Verweildauer (T_{L}) erreicht hat.
